(19) Europäisches Patentamt
European Patent Office
Office européen des brevets

(11) Veröffentlichungsnummer : **0 011 188**
**B1**

(12) **EUROPÄISCHE PATENTSCHRIFT**

(45) Veröffentlichungstag der Patentschrift : **28.04.82**

(21) Anmeldenummer : **79104250.0**

(22) Anmeldetag : **31.10.79**

(51) Int. Cl.³ : **C 07 C148/00**, C 07 C120/00, C 07 C149/415, C 07 C149/42, C 07 C121/75

# (54) Verfahren zur Herstellung von Cyanoaryläthern und Cyanoarylthioäthern.

(30) Priorität : **08.11.78 DE 2848365**

(43) Veröffentlichungstag der Anmeldung : **28.05.80 (Patentblatt 80/11)**

(45) Bekanntmachung des Hinweises auf die Patenterteilung : **28.04.82 Patentblatt 82/17**

(84) Benannte Vertragsstaaten : **BE CH DE FR GB**

(56) Entgegenhaltungen :
**JOURNAL OF THE CHEMICAL SOCIETY (C), 1971, London**
**D.E.L. CARRINGTON et al. « 1,2-Benzisothiazoles. Part. I. Reaction of 3-Chloro-1,2-benzisothiazole with Nucleophiles »**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17 e**
**D-6710 Frankenthal (DE)**
Erfinder : **Markert, Juergen, Dr.**
**Bruesseler Ring 38**
**D-6700 Ludwigshafen (DE)**
Erfinder : **Merger, Franz, Dr.**
**Max-Slevogt-Strasse 25**
**D-6710 Frankenthal (DE)**
Erfinder : **Towae, Friedrich, Dr.**
**Parkstrasse 22**
**D-6700 Ludwigshafen (DE)**

**EP 0 011 188 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Cyanoaryläthern und Cyanoarylthioäthern

Die Erfindung betrifft ein neues Verfahren zur Herstellung von Cyanoaryläthern und Cyanoarylthioäthern durch Umsetzung von Benzisothiazolen mit Diaralkylcarbonaten oder Dialkylcarbonaten bei erhöhter Temperatur.

Es ist aus J. Chem Soc. 1971, Seiten 3 262 bis 3 265 bekannt, daß 3-Chlorbenzisothiazole unter dem Einfluß von Cyanidionen teilweise o-Thiocyanatobenzonitrile neben Bis-(o-cyanodiphenyl)-disulfiden bilden. Diese Stoffe können reduktiv in die entsprechenden o-Cyanophenylmercaptane überführt werden. Die o-Cyanophenylmercaptane können mit Estern starker organischer Säuren wie Methyljodid oder Dimethylsulfat in die entsprechenden 2-Cyanomethylthiobenzole umgewandelt werden (Houben-Weyl, Methoden der Organischen Chemie, Band 9, Seiten 112 bis 116).

Diese Reaktionsschritte sind nun mit erheblichen Nachteilen verbunden. Die Ringöffnungsreaktion bei Benzisothiazolen ist uneinheitlich. Bei der Methylierung der o-Cyanophenylmercaptane mit Methyljodid oder Dimethylsulfat fällt in stöchiometrischen Mengen anorganisches Salz an, dessen Beseitigung erhebliche Kosten verursacht. Auch sind wegen der hohen Toxizität der verwendeten Methylierungsmittel strenge Sicherheitsmaßnahmen zu beachten. Das Verfahren ist im Hinblick auf Ausbeute und Reinheit des Endstoffes, einfachen und wirtschaftlichen Betrieb sowie Betriebssicherheit und Umweltschutz unbefriedigend.

Es wurde nun gefunden, daß man Cyanoaryläther und Cyanoarylthioäther der Formel

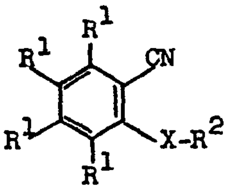

(I)

worin die einzelnen Reste $R^1$ gleich oder verschieden sein können und jeweils ein Wasserstoffatom, einen aliphatischen, cycloaliphatischen, araliphatischen, aromatischen Rest, ein Halogenatom, eine Cyangruppe, eine Nitrogruppe, den Rest $R^3$—O—, den Rest

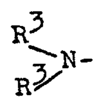

bedeuten, darüber hinaus auch zwei benachbarte Reste $R^1$ für Glieder eines anellierten Benzolkernes stehen können, $R^2$ einen aliphatischen oder araliphatischen Rest bezeichnet, $R^3$ für ein Wasserstoffatom, einen aliphatischen, cycloaliphatischen, araliphatischen oder aromatischen Rest steht, X ein Schwefelatom oder ein Sauerstoffatom bedeutet, vorteilhaft erhält, wenn man Benzisothiazole der Formel

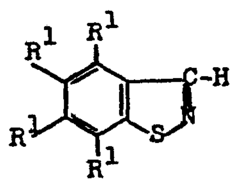

(II)

worin $R^1$ die vorgenannte Bedeutung besitzt, mit diaraliphatischen Carbonaten oder dialiphatischen Carbonaten der Formel

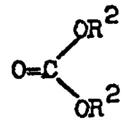

(III)

worin die einzelnen Reste $R^2$ gleich oder verschieden sein können und die vorgenannte Bedeutung besitzen, bei einer Temperatur oberhalb 100 °C umsetzt, wobei beiläufige Reaktionen auftreten können.

Die Umsetzung kann für den Fall der Verwendung von Benzisothiazol bzw. 5-Nitro-benzisothiazol und Dimethylcarbonat durch die folgenden Formeln wiedergegeben werden :

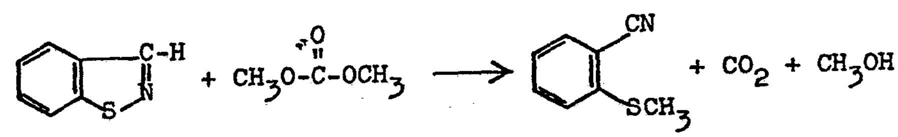

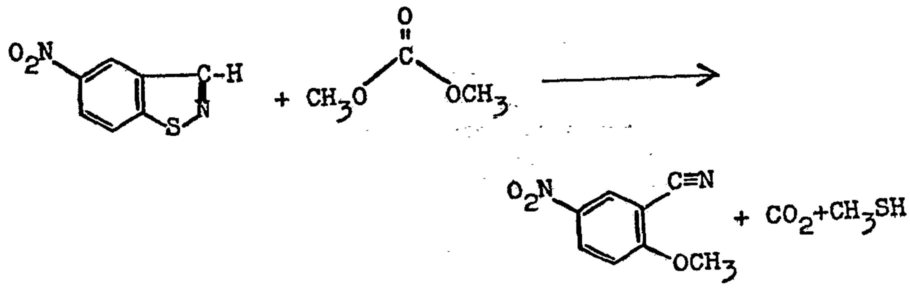

Im Vergleich zu dem bekannten Verfahren liefert das Verfahren nach der Erfindung auf einfacherem und wirtschaftlicherem Wege Cyanoaryläther und -thioäther in besserer Ausbeute und Reinheit. Umständliche Abtrennungs- und Neutralisationsoperationen sowie die Bildung störender Nebenstoffe wie Salze werden vermieden. Außer dem Endstoff werden lediglich ein Mol Alkohol oder Thioalkohol sowie das nicht toxische Kohlendioxid erhalten; das erfindungsgemäße Verfahren ist somit umweltfreundlicher als das bekannte Verfahren. Der freiwerdende Alkohol kann in bekannter Weise ohne Phosgen wieder mit CO und Sauerstoff zu Dialkylcarbonat bzw. Diaralkylcarbonat

$$2\,R^2OH + CO + 1/2\,O_2 \rightarrow O = C\,(OR^2)_2 + H_2O$$

verarbeitet werden (deutsche Offenlegungsschrift 23 34 736). Alle diese vorteilhaften Eigenschaften des erfindungsgemäßen Verfahrens sind im Hinblick auf den Stand der Technik überraschend.

Die Dialkylcarbonate können in bekannter Weise, z.B. nach den in der deutschen Offenlegungsschrift 21 60 111 angegebenen Verfahren oder zweckmäßiger, insbesondere aus Gründen des Umweltschutzes, durch Umsetzung eines Alkohols mit Kohlenmonoxid und Sauerstoff in Gegenwart von Kupferkatalysatoren, nach dem in der deutschen Offenlegungsschrift 23 34 736 beschriebenen Verfahren, hergestellt werden. Bevorzugte Ausgangsstoffe II und III und dementsprechend bevorzugte Endstoffe I sind solche, in deren Formeln die einzelnen Reste $R^1$ gleich oder verschieden sein können und jeweils ein Wasserstoffatom, einen Alkylrest mit 1 bis 7, zweckmäßig 1 bis 4 Kohlenstoffatomen, einen Cyclohexylrest, einen Phenylrest, einen Aralkylrest mit 7 bis 12 Kohlenstoffatomen, ein Bromatom, ein Fluoratom, ein Chloratom, eine Cyangruppe, eine Nitrogruppe, den Rest $R^3$—O—, den Rest

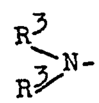

bedeuten, darüber hinaus auch zwei benachbarte Reste $R^1$ für Glieder eines anellierten Benzolkernes stehen, die einzelnen Reste $R^2$ gleich oder verschieden sein können und jeweils einen Aralkylrest mit 7 bis 12 Kohlenstoffatomen oder insbesondere einen Alkylrest mit 1 bis 7, zweckmäßig 1 bis 4 Kohlenstoffatomen bezeichnen, die einzelnen Reste $R^3$ gleich oder verschieden sein können und jeweils für ein Wasserstoffatom, einen Phenylrest, einen Aralkylrest mit 7 bis 12 Kohlenstoffatomen, einen Cyclohexylrest oder insbesondere einen Alkylrest mit 1 bis 12, zweckmäßig 1 bis 6 Kohlenstoffatomen, stehen, X ein Schwefelatom oder ein Sauerstoffatom bedeutet. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Es kommen als Ausgansstoffe II beispielsweise in Frage: Benzisothiazol; 1,2-Naphthisothiazol, 2,3-Naphthisothiazol, 3,4-Naphthisothiazol; in 4-Stellung, 5-Stellung, 6-Stellung und/oder 7-Stellung einfach, zweifach, dreifach oder vierfach mit gleichen oder verschiedenen Substituenten durch die Methyl-, Äthyl-, n-Propyl-, Isopropyl-, n-Butyl-, Isobutyl-, sek.-Butyl-, tert.-Butyl-, Cyclohexyl-, Benzyl-, Phenyl-, Cyan-, Nitro-, Brom-, Fluor-, Chlor-, Hydroxy-, Amino-, Methylamino-, Äthylamino-, Propylamino-, Isopropylamino-, Butylamino-, sek.-Butylamino-, tert.-Butylamino-, Isobutylamino-, Cyclohexylamino-, Benzylamino-, Phenylamino-, Dimethylamino-, Diäthylamino-, Dipropylamino-, Diisopropylamino-, Dibutylamino-, Di-sek.-butylamino-, Di-tert.-butylamino-, Diisobutylamino-, Dicyclohexylamino-, Dibenzylamino-, Diphenylamino-, Methoxy-, Äthoxy-, Propoxy-, Isopropoxy-, Butoxy-, Isobutoxy-, sek.-Butoxy, tert.-Butoxy-gruppe substituiertes Benzisothiazol; entsprechende, in 1,2-, 2,3- oder 3,4-Stellung oder jeweils in den 1-, 2-, 3- oder 4-Stellungen durch vorgenannte Gruppen einfach oder zweifach substituierte 1,2-, 2,3- oder 3,4-Naphthisothiazole

(
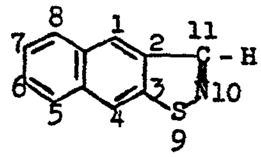
2,3-Naphthisothiazol).

Als Ausgangsstoffe III kommen in Betracht: Dimethylcarbonat, Diäthylcarbonat, Di-n-propylcarbonat, Di-n-butylcarbonat, Diisobutylcarbonat, Dibenzylcarbonat, Methyläthylcarbonat, Methylpropylcarbonat, Äthylpropylcarbonat; bevorzugt sind Dimethylcarbonat, Diäthylcarbonat, Di-n-propylcarbonat, Methyläthylcarbonat, Dibenzylcarbonat.

Im allgemeinen erhält man bei der Reaktion Gemische der entsprechend substituierten 2-Cyano-1-mercapto-benzole und 2-Cyano-1-oxy-benzole bzw. Gemische der entsprechenden Cyanomercapto- und Cyanooxy-naphthaline. Je nach Wahl der Konstitution des Ausgangsstoffes, der Temperatur und gegebenenfalls des Lösungsmittels fällt die eine oder andere Gemischkomponente als Nebenprodukt oder sogar nur noch in praktisch vernachlässigbarer Menge an. Nitrogruppen-haltige Ausgangsstoffe II werden in der Regel leichter zu Cyanoaryläthern I umgesetzt. Geht man von Ausgangsstoffen aus, die ein oder mehrere Reste $R^3$ in der Bedeutung Wasserstoff besitzen, so kann man durch Erhöhung der Temperatur und/oder der Menge an Ausgangsstoff III neben der Bildung der Gruppe $—X—R^2$ im Endstoff I noch eine Umwandlung der Hydroxygruppen, Aminogruppen und monosubstituierten Aminogruppen des Ausgangsstoffes II in Äther, monosubstituierte Aminogruppen und disubstituierte Aminogruppen im Endstoff I erzielen; der Grad der Substituierung kann durch entsprechende Steuerung der Temperatur und der Gewichtsverhältnisse der Reaktionspartner sowie der Reaktionszeit beliebig eingestellt werden, wobei die geeigneten Bedingungen durch wenige Testversuche leicht festzustellen sind. Die Umsetzung wird bei einer Temperatur oberhalb 100 °C, im allgemeinen zwischen 100 und 350 °C, vorzugsweise von 110 bis 250 °C, im Falle der Herstellung von Cyano-thioäthern I vorteilhaft von 110 bis 230 °C, insbesondere von 130 bis 220 °C; im Falle der Herstellung von Cyanoätherverbindungen I, ausgehend von nitrogruppenhaltigen Verbindungen II vorteilhaft von 110 bis 250 °C, insbesondere von 130 bis 250 °C, ausgehend von nitrogruppenfreien Verbindungen II vorteilhaft von 180 bis 250 °C, insbesondere von 210 bis 250 °C, durchgeführt. Bevorzugt sind auch die in folgender Tabelle aufgeführten Temperaturintervalle.

(Siehe die Tabelle Seite 5)

Tabelle

| Ausgangsstoff II enthält eine oder mehrere: eine oder mehrere Hydroxygruppen | Aminogruppen | monosubstituierte Aminogruppen | Der Endstoff enthält: Äthergruppen | monosubstituierte Aminogruppen | disubstituierte Aminogruppen | Struktur des Endstoffes | Reaktionstemperaturintervall von .... bis .... °C |
|---|---|---|---|---|---|---|---|
| + | | | + | | | Thioäther I | 130 - 200 |
| | + | | | + | | " | 130 - 170 |
| | + | | | | + | " | 170 - 210 |
| | | + | | | + | " | 160 - 210 |
| + | + | | + | + | | " | 150 - 170 |
| + | + | | + | | + | " | 170 - 220 |
| + | | | + | | | nitrogruppenhaltige Äther I | 130 - 250 |
| | + | | | + | | " | 130 - 160 |
| | + | | | | + | " | 160 - 250 |
| | | + | | | + | " | 150 - 250 |
| + | + | | + | + | | " | 130 - 170 |
| + | + | | + | | + | " | 170 - 250 |
| + | | | + | | | nitrogruppenfreie Äther I | 210 - 250 |
| | + | | | | + | " | 220 - 250 |
| | | + | | | + | " | 220 - 250 |
| + | + | | + | | + | " | 220 - 250 |

Die Ausgangsstoffe II werden mit den Ausgangsstoffen III in stöchiometrischer Menge oder im Überschuß oder Unterschuß umgesetzt, vorteilhaft im Falle der Umsetzung von hydroxy- und aminofreien

Verbindungen II von 1 bis 10 Mol, insbesondere von 1,2 bis 5 Mol Ausgangsstoff III je Mol Ausgangsstoff II oder bei zusätzlicher Umsetzung von Hydroxy- und/oder Aminogruppen im Falle der Umsetzung von monosubstituierten Aminoverbindungen II oder Hydroxyverbindungen II zu disubstituierten Aminoverbindungen I oder Ätherverbindungen I von 2 bis 50, insbesondere von 2,5 bis 20 Mol Ausgangsstoff III je Mol Ausgangsstoff II oder im Falle der Umsetzung von unsubstituierten Aminoverbindungen II zu disubstituierten Aminoverbindungen I von 3 bis 50, insbesondere von 3,5 bis 20 Mol Ausgangsstoff III je Mol Ausgangsstoff II oder im Falle der Umsetzung von unsubstituierten Aminoverbindungen II zu monosubstituierten Aminoverbindungen I von 0,5 bis 3, insbesondere von 1 bis 2 Mol Ausgangsstoff III je Mol Ausgangsstoff II. Die Umsetzung erfolgt im allgemeinen drucklos oder unter Druck, vorzugsweise unter dem im Autoklaven bei vorgenannten Temperaturen sich einstellenden Dampfdruck des Reaktionsgemisches, zweckmäßig von 1 bis 250 bar, kontinuierlich oder diskontinuierlich. Zweckmäßig dient das Reaktionsgemisch gleichzeitig als Lösungsmedium bzw. Suspensionsmedium. In solchen Fällen ist es bisweilen vorteilhaft, einen Überschuß an Ausgangsstoff III und/oder schon am Anfang eine Zusatzmenge von dem bei der Reaktion sich bildenden Alkohol oder Thioalkohol zuzugeben. Gegebenenfalls können unter den Reaktionsbedingungen inerte Lösungsmittel verwendet werden. Als Lösungsmittel kommen z.B. in Frage : aromatische Kohlenwasserstoffe, z.B. Toluol, Äthylbenzol, o-, m-, p-Xylol, Isopropylbenzol, Methylnaphthalin, Chlorbenzol, o- und m-Dichlorbenzol, o-, m-, p-Chlortoluol, 1,2,4-Trichlorbenzol ; Äther, z.B. Äthylpropyläther, Methyltert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan ; aliphatische oder cycloaliphatische Kohlenwasserstoffe, z.B. Heptan, Nonan, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan ; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, insbesondere solche mit 1 bis 4 Kohlenstoffatomen ; und entsprechende Gemische. Bevorzugt ist Methanol. Zweckmäßig verwendet man das Lösungsmittel in einer Menge von 100 bis 2 000 Gewichtsprozent, bezogen auf Ausgangsstoff III.

Man verwendet gegebenenfalls als Katalysator ein tertiäres Amin, vorteilhaft in einer Menge von 0,1 bis 10, insbesondere von 1 bis 5 Molprozent, bezogen auf Ausgangsstoff II. Auch Gemische der genannten Katalysatoren kommen für die Reaktion in Betracht. Das Amin kann auch in Gestalt von Diaminen, entsprechenden Salzen oder einem quaternären Salz verwendet werden. Geeignete Katalysatoren sind Trimethylamin, Dimethylamino-neopentanol, N,N'-Tetramethyl-diamino-neopentan, Äthyldimethylamin, Lauryldimethylamin, Stearyldimethylamin, Pyridin, α-, β- und γ-Picolin, Chinolin, Isochinolin, Chinazolin, Chinoxalin, Amyldimethyl-amin, Propyldimethylamin, Butyldimethylamin, N-Methylimidazol, N-Methylpyrrol, 2,6- und 2,4-Lutidin, Triäthylendiamin, p-Dimethylaminopyridin, N,N-Dimethylcyclohexylamin, Pyrimidin, Acridin ; Di-(methyl)-, Di-(äthyl)-, Di-(n-propyl)-, Di-(n-butyl)-, Di-(pentyl)-, Di(n-hexyl)-, Di-(n-heptyl)-, Di-(n-octyl)-, Di-(n-nonyl)-, Di-(n-decyl)-anilin und mit vorgenannten Resten am Stickstoffatom einfach substituiertes Pyrazolidin, Imidazolidin, Morpholin, Piperidin, Pyrrolidin ; entsprechende Katalysatoren mit 2 oder 3 vorgenannten, aber unterschiedlichen Resten, z.B. Dimethyläthylamin. Ebenfalls kommen auch tertiäre Aminogruppen enthaltende Polymere, z.B. 4-Polyvinylpyridin und Polyvinylimidazol-(N), in Frage.

Besonders vorteilhaft sind Trimethylamin, Pyridin, γ-Picolin, 4-Pyrrolidino-(1')-pyridin und andere Pyridinderivate wie o-Methyl-, m-Methyl-, o-Äthyl-, m-Äthyl-, p-Äthyl-, o-Propyl-, m-Propyl-, p-Propylpyridin, Dimethylamino-neopentanol, N,N'-Tetramethyldiamino-neopentan, p-Methoxy-, p-Äthoxy, p-Propoxy-pyridin, p-Dimethylaminopyridin, p-Diäthylaminopyridin, p-Dipropylaminopyridin, 4-Polyvinylpyridin, N-Methylimidazol.

Die Umsetzung kann wie folgt durchgeführt werden : Ein Gemisch der Ausgangsstoffe II, III und gegebenenfalls des Katalysators und des Lösungsmittels wird während 1 bis 20 Stunden bei der Reaktionstemperatur gehalten. Aus dem Gemisch wird dann der Endstoff in üblicher Weise, z.B. durch fraktionierte Destillation, abgetrennt.

Die nach dem Verfahren der Erfindung herstellbaren Aralkylaryl- und Alkylaryl-cyano-thioäther und -äther sind wertvolle Ausgangsstoffe für die Herstellung von Farbstoffen, Pflanzenschutzmitteln und Riechstoffen. Bezüglich der Verwendung wird auf vorgenannte Veröffentlichungen und Ullmanns Encyklopädie der technischen Chemie, Band 13, Seiten 450 bis 453, und Band 14, Seiten 760 bis 763, verwiesen.

Die in den folgenden Beispielen aufgeführten Teile bedeuten Gewichtsteile.

## Beispiel 1

15 Teile 5-Aminobenzisothiazol werden mit 18 Teilen Dimethylcarbonat in 100 Teilen Dioxan gelöst und auf 160 °C erhitzt. Nach 3 Stunden werden das Lösungsmittel und gebildetes Methanol abdestilliert und der Rückstand mit Äther ausgewaschen. Man erhält 5,6 Teile (68 % der Theorie) 2-Cyano-4-methylamino-1-methylthiobenzol vom Fp 84 bis 88 °C. Der Umsatz beträgt 46 Prozent.

Beispiel 2

200 Teile 5-Aminobenzisothiazol werden mit 600 Teilen Dimethylcarbonat und 10 Teilen N-Methylimidazol als Katalysator 10 Stunden auf 200 °C gehalten. Nach beendeter Reaktion werden überschüssiges Dimethylcarbonat und Methanol abgezogen und der Rückstand destilliert. Man erhält 175 Teile (71 % der Theorie) 2-Cyano-4-dimethylamino-1-methylthiobenzol vom Fp 44 bis 46 °C. Der Umsatz beträgt 96 Prozent.

Beispiel 3

25 Teile 5-Nitrobenzisothiazol werden mit 63 Teilen Dimethylcarbonat 10 Stunden auf 200 °C gehalten. Nach beendeter Reaktion werden überschüssiges Dimethylcarbonat und gebildetes Methanol abgezogen und der Rückstand mit Äther versetzt. Man erhält 15,7 Teile (87 % der Theorie) 2-Cyano-1-methoxy-4-nitrobenzol vom Fp 111 °C. Der Umsatz beträgt 73 Prozent.

Beispiel 4

10 Teile Benzisothiazol werden mit 66 Teilen Dimethylcarbonat und 0,5 Teilen N-Methylimidazol als Katalysator 5 Stunden auf 200 °C gehalten. Nach beendeter Reaktion werden überschüssiges Dimethylcarbonat und Methanol abgezogen und der Rückstand destilliert. Man erhält 10,3 Teile (93 % der Theorie) 2-Cyano-1-methylthiobenzol vom Kp 106 °C (0,35 mbar). Der Umsatz ist praktisch quantitativ.

Beispiel 5

200 Teile 4,7-Diaminobenzisothiazol werden mit 1 300 Teilen Dimethylcarbonat 5 Stunden auf 220 °C gehalten. Nach beendeter Reaktion werden überschüssiges Dimethylcarbonat und Methanol abgezogen und der Rückstand destilliert. Man erhält 80 Teile (36 % der Theorie) 2-Cyano-3,6-bis(dimethylamino)-1-methylthiobenzol vom Kp 205 bis 210 °C (1,5 mbar). Der Umsatz beträgt 78 Prozent.

Beispiel 6

30 Teile 4-Chlorbenzisothiazol werden mit 80 Teilen Dimethylcarbonat und 1,5 Teilen N-Methylimidazol als Katalysator 5 Stunden auf 190 °C gehalten. Nach beendeter Reaktion werden überschüssiges Dimethylcarbonat und Methanol abdestilliert und der Rückstand destilliert. Man erhält 27,1 Teile (97 % der Theorie) 3-Chlor-2-cyano-1-methylthiobenzol vom Fp 91 °C. Der Umsatz beträgt 86 Prozent.

Beispiel 7

30 Teile 1,2-Naphthisothiazol werden mit 66 Teilen Dimethylcarbonat und 1,5 Teilen N-Methylimidazol als Katalysator 20 Stunden auf 210 °C gehalten. Nach beendeter Reaktion werden überschüssiges Dimethylcarbonat und Methanol abdestilliert und der Rückstand destilliert. Man erhält 18 Teile (96 % der Theorie) 2-Cyano-1-methylthionaphthalin vom Kp 135 bis 139 °C (0,3 mbar). Der Umsatz beträgt 58 Prozent.

**Anspruch**

Verfahren zur Herstellung von Cyanoaryläthern und Cyanoarylthioäthern der Formel

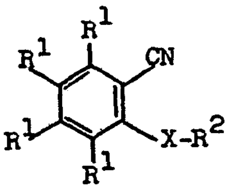

(I)

worin die einzelnen Resten $R^1$ gleich oder verschieden sein können und jeweils ein Wasserstoffatom, einen aliphatischen, cycloaliphatischen, araliphatischen, aromtischen Rest, ein Halogenatom, eine Cyangruppe, eine Nitrogruppe, den Rest $R^3$—O—, den Rest

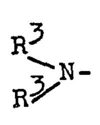

bedeuten, darüber hinaus auch zwei benachbarte Reste $R^1$ für Glieder eines anellierten Benzolkernes stehen können, $R^2$ einen aliphatischen oder araliphatischen Rest bezeichnet, $R^3$ für ein Wasserstoffatom, einen aliphatischen, cycloaliphatischen, araliphatischen oder aromatischen Rest steht, X ein Schwefelatom oder ein Sauerstoffatom bedeutet, dadurch gekennzeichnet, daß man Benzisothiazole der Formel

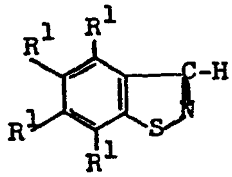

(II)

worin $R^1$ die vorgenannte Bedeutung besitzt, mit diaraliphatischen Carbonaten oder dialiphatischen Carbonaten der Formel

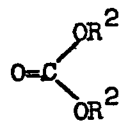

(III)

worin die einzelnen Reste $R^2$ gleich oder verschieden sein können und die vorgenannte Bedeutung besitzen, bei einer Temperatur oberhalb 100 °C umsetzt, wobei beiläufige Reaktionen auftreten können.

**Claim**

A process for the production of cyanoaryl ethers or cyanoaryl thioethers of the formula

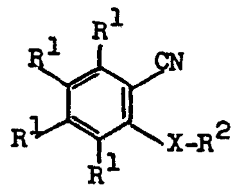

(I)

where the individual radicals $R^1$ may be identical or different and each denotes hydrogen, an aliphatic, cycloaliphatic, araliphatic or aromatic radical, halogen, cyano, nitro, $R^3$—O— or

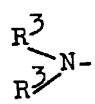

and in addition two adjacent $R^1$'s may be members of a fused benzene nucleus, $R^2$ denotes an aliphatic or araliphatic radical, $R^3$ is hydrogen, an aliphatic, cycloaliphatic, araliphatic or aromatic radical, and X is sulphur or oxygen, characterized in that a benzisothiazole of the formula

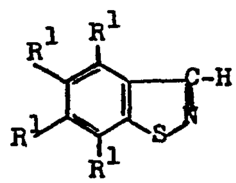

(II)

where $R^1$ has the above meaning, is reacted with a diaraliphatic carbonate or a dialiphatic carbonate of the formula

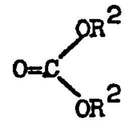

(III)

where the individual radicals $R^2$ may be identical or different and have the above meaning, at a temperature above 100 °C, it being possible for side reactions to occur.

**Revendication**

Procédé de préparation d'éthers cyanaryliques et de thioéthers cyanaryliques de formule

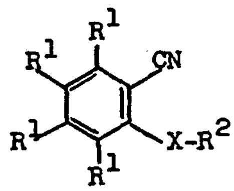

(I)

dans laquelle les divers symboles $R^1$, ayant des significations identiques ou différentes, représentent chacun un atome d'hydrogène, un groupe aliphatique, cycloaliphatique, araliphatique, aromatique, un atome d'halogène, un groupe cyano, un groupe nitro, le groupe $R^3$—O—, le groupe

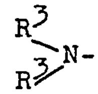

deux symboles $R^1$ voisins pouvant en outre représenter des chaînons d'un cycle benzénique condensé, $R^2$ représente un reste aliphatique ou araliphatique, $R^3$ représente un atome d'hydrogène, un reste aliphatique, cycloaliphatique, araliphatique ou aromatique, X représente un atome de soufre ou un atome d'oxygène, caractérisé en ce que l'on fait réagir à une température supérieure à 100 °C des benzoisothiazoles de formule

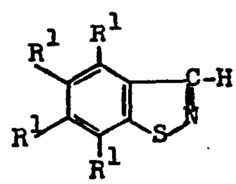

(II)

dans laquelle $R^1$ a la signification indiquée ci-dessus, avec des carbonates diaraliphatiques ou des carbonates dialiphatiques de formule

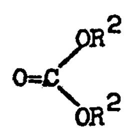

(III)

dans laquelle les divers symboles $R^2$, qui peuvent être identiques ou différents, ont la signification indiquée ci-dessus, la réaction pouvant s'accompagner de réactions secondaires.